# EUROPEAN PATENT SPECIFICATION

(11) **EP 3 145 391 B1**
(45) Date of publication and mention of the grant of the patent: **01.11.2023**
(21) Application number: 15796515.3
(22) Date of filing: 21.05.2015
(51) Int. Cl.: A61B 5/00, G01N 33/487, H01M 10/44, H02J 7/04, F04B 49/06, F04B 51/00, G01K 13/00, G01N 33/66, H01M 10/46

(54) **HANDHELD ANALYTE METER WITH RECHARGING CONTROL FOR IMPROVED ANALYTE TESTING**
TRAGBARES ANALYTMESSGERÄT MIT WIEDERAUFLADUNGSSTEUERUNG FÜR VERBESSERTE ANALYTTESTS
DISPOSITIF DE MESURE DE SUBSTANCE À ANALYSER PORTABLE AYANT UNE COMMANDE DE RECHARGE POUR UNE ANALYSE DE SUBSTANCE À ANALYSER AMÉLIORÉE

(30) Priority: 22.05.2014 US 201414285144
(43) Date of publication of application: 29.03.2017
(73) Proprietor: F. Hoffmann-La Roche AG, 4070 Basel (CH); Roche Diabetes Care GmbH, 68305 Mannheim (DE)
(72) Inventor: RAMEY, Blaine, Indianapolis, Indiana 46204 (US); SIMPSON, Joseph, Fishers, Indiana 46037 (US)
(74) Representative: Altmann Stößel Dick Patentanwälte PartG mbB
(86) International application number: PCT/US2015/031979
(87) International publication number: WO 2015/179642

(56) References cited:
- EP-A1- 2 584 666
- US-A1- 2011 279 092
- US-A1- 2012 095 312
- US-A1- 2013 006 536
- US-A1- 2013 187 614
- US-B2- 8 164 468

## Description

### CROSS-REFERENCE TO RELATED APPLICATIONS

This patent application claims priority to and the benefit of US Patent Application No. 14/285,144 (filed 22 May 2014).

### TECHNICAL FIELD

This disclosure relates generally to engineering and medical diagnostics, and more particularly, it relates to handheld *in vitro* analyte meters having a rechargeable battery and recharging controls, such as a blood glucose meter or an infusion pump controller with an integral blood glucose meter.

### BACKGROUND

Diabetes mellitus, often referred to as diabetes, is a chronic condition in which a person has elevated blood glucose levels that result from the body's inability to produce insulin, use insulin or both. There are three (3) main types of diabetes. First is Type 1 diabetes, which usually strikes children and young adults and is linked to conditions such as autoimmune, genetic, environmental or a combination. Second is Type 2 diabetes, which accounts for 90%-95% of diabetes cases and which is linked to obesity and physical inactivity. Lastly is gestational diabetes, which is a form of glucose intolerance diagnosed during pregnancy, and which usually resolves itself soon after delivery.

In 2013, some 382 million people worldwide were estimated to have diabetes, and an estimated 5.1 million people between the ages of 20 and 79 die from diabetes annually, according to the Int'l Diabetes Foundation Diabetes Atlas. In the US, nearly 24 million Americans have diabetes with an estimated 25% of seniors age 60 and older being affected, according to The Centers for Disease Control and Prevention. Diabetes costs are estimated to be $174 billion in the US alone every year, according to the National Diabetes Information Clearinghouse. Without treatment, diabetes can lead to severe complications such as heart disease, stroke, blindness, kidney failure, amputations and even death related to pneumonia and flu.

Handheld blood glucose meters are used by persons with diabetes to measure blood glucose using a test element/test strip, such as a biosensor, inserted into the meter. The test element has a collection area on one end that extends from the meter. A small drop of blood is placed on the collection area to initiate an electrochemical or photometric enzymatic reaction to determine a blood glucose measurement. This enzymatic reaction is sensitive to temperature, so an acceptable temperature range is established for performing accurate blood glucose measurements. Prior to initiating a blood glucose measurement, a temperature sensor typically inside the meter housing measures temperature to determine whether the temperature is within the acceptable temperature range. If the temperature is within the acceptable temperature range, a glucose measurement is performed. If the temperature is outside the acceptable temperature range, a glucose measurement is not performed.

As handheld blood glucose meters have increased in capability with features such as color displays, multiple microprocessors and wireless communications, power consumption also has increased, necessitating a use of rechargeable batteries. During recharging, the charging current and electrochemical reactions cause the battery to self-heat or warm, which causes a temperature rise inside the housing or self-heating, particularly during the first hour or two of recharging. When the temperature inside the blood glucose meter rises, the meter's estimate for the acceptable temperature range to perform a blood glucose test can be inaccurate. Modulating charging current during a recharging session can create self-heating thermal conditions that are difficult to predict. An inaccurate temperature measurement can cause a lock-out condition that prevents a blood glucose measurement from being performed or mask a lock-out condition that inappropriately allows a blood glucose measurement to be performed, potentially for the first hour of recharging. Examples of rechargeable handheld analyte meters include LifeScan's OneTouch Verio IQ^{®} Blood Glucose Monitoring System; Bayer's Contour^{®} USB Glucose Monitoring System; and Roche Diagnostics' Accu-Chek^{®} Inform II Blood Glucose Monitoring System.

What is needed is a rechargeable analyte meter with recharging control to manage battery self-heating for improved analyte testing.

### BRIEF SUMMARY

An inventive concept described herein includes providing recharging controls to manage rechargeable battery self-heating in handheld analyte meters for improved analyte testing. Advantageously, the recharging controls address the problem of self-heating thermal conditions that can occur when modulating charging current during a recharging session and that are difficult to predict. Such recharging controls therefore attenuate instances when a false lock-out condition prevents a blood glucose measurement from being performed or mask a lock-out condition that inappropriately allows a blood glucose measurement to be performed. This inventive concept is achieved by selecting a maximum charging current prior to the beginning of a charging session, which does not change during the charging session. The maximum charging current is based upon a capacity of a charging source and a first temperature compared to a first temperature range, where the first temperature closely reflects a meter temperature as measured by a first sensor. Prior to initiating an analyte measurement, a second sensor measures a second temperature that more closely reflects the reaction temperature near a test element, which is compared to a high-lockout temperature range and/or a low-lockout temperature range. In this manner, the second temperature sensor more closely reflects the test element temperature, while the first temperature sensor more closely reflects the battery temperature. This inventive concept therefore can be incorporated into exemplary devices and methods as described herein and in more detail below.

For example, and in one embodiment, handheld analyte meters are provided, such as a blood glucose meter or a combination blood glucose meter and infusion pump controller, with a recharging control that improves analyte testing by regulating self-heating that occurs during recharging and that can adversely influence analyte tests. Briefly, the meters can include a housing, a cable connector, a rechargeable battery, a main processor, a display, a first temperature sensor, a measurement module, a second temperature sensor, a battery charger, and a charging control. A maximum charging current is selected prior to initiating a charging session, and the maximum charging current does not change during the charging session. The maximum charging current is selected based upon a capacity of a charging source and a first temperature compared to a first temperature range. By selecting the maximum charging current in this manner, the risk of the measurement module preventing a test under high lock-out conditions and the risk of the measurement module allowing a test when low lock-out conditions are masked is attenuated or reduced.

In view of the foregoing, and in another embodiment, methods are provided for controlling handheld analyte meter recharging, such as a blood glucose meter or a combination blood glucose meter and infusion pump controller, which improve analyte testing by regulating self-heating that occurs during recharging and can adversely influence analyte tests. Briefly, the methods of controlling handheld meter recharging can include connecting a cable to a charging source and to the handheld meter, identifying a charging source capacity, measuring a first temperature inside the meter with a first temperature sensor, selecting a maximum charging current, and recharging a rechargeable battery. The maximum charging current is selected prior to initiating a charging session, and the maximum charging current does not change during the charging session. The maximum charging current is selected based upon the charging source capacity and a first temperature compared to a first temperature range. By selecting the maximum charging current in this manner, the risk of the measurement module preventing a test under high lock-out conditions and the risk of the measurement module allowing a test under low lock-out conditions that are masked is reduced.

These and other advantages, effects, features and objects of the inventive concept will become better understood from the description that follows. In the description, reference is made to the accompanying drawings, which form a part hereof and in which there is shown by way of illustration, not limitation, embodiments of the inventive concept.

### BRIEF DESCRIPTION OF THE DRAWINGS

The advantages, effects, features and objects other than those set forth above will become more readily apparent when consideration is given to the description of exemplary embodiments below. Such description makes reference to the following drawings, wherein:
Fig. 1 shows a person with diabetes and a health care provider in a clinical environment.
Fig. 2 shows a person with diabetes with a variety of diabetes devices.
Fig. 3 shows a perspective view of a handheld analyte meter with a rechargeable battery.
Fig. 4 shows a perspective cut-away view of a handheld analyte meter with a rechargeable battery.
Fig. 5 shows a printed circuit board for a handheld analyte meter with a rechargeable battery.
Fig. 6 shows a block diagram of a handheld analyte meter with a rechargeable battery.
Fig. 7 shows a block diagram of a charging module including a battery charger and charging control program for a handheld analyte meter with a rechargeable battery.
Fig. 8 shows a flowchart of a charge control program for selecting a charge current for the battery charger.
Fig. 9 shows a flowchart of a method of recharging a handheld analyte meter with a rechargeable battery.
Fig. 10 shows test results from a heated environmental chamber comparing a handheld analyte meter with a charging control and a handheld analyte meter without charging control during recharging.
Fig. 11 shows test results from a cooled environmental chamber comparing a handheld analyte meter with a charging control and a handheld analyte meter without charging control during recharging.

While the inventive concept is susceptible to various modifications and alternative forms, exemplary embodiments thereof are shown by way of example in the drawings and are herein described in detail. It should be understood, however, that the description of exemplary embodiments that follows is not intended to limit the inventive concept to the particular forms disclosed, but on the contrary, the intention is to cover all advantages, effects, features and objects falling within the scope as defined by the claims below. Reference should therefore be made to the embodiments described herein and claims below for interpreting the scope of the inventive concept. As such, it should be noted that the embodiments described herein may have advantages, effects, features and objects useful in solving other problems.

### DESCRIPTION OF EXEMPLARY EMBODIMENTS

The devices and methods now will be described more fully hereinafter with reference to the accompanying drawings, in which some, but not all embodiments of the inventive concept are shown. Indeed, the devices and methods may be embodied in many different forms and should not be construed as limited to the embodiments set forth herein; rather, these embodiments are provided so that this disclosure will satisfy applicable legal requirements.

Likewise, many modifications and other embodiments of the devices and methods described herein will come to mind to one of skill in the art to which the disclosure pertains having the benefit of the teachings presented in the foregoing descriptions and the associated drawings. Therefore, it is to be understood that the devices and methods are not to be limited to the specific embodiments disclosed and that modifications and other embodiments are intended to be included within the scope of the appended claims. Although specific terms are employed herein, they are used in a generic and descriptive sense only and not for purposes of limitation.

Unless defined otherwise, all technical and scientific terms used herein have the same meaning as commonly understood by one of skill in the art to which the disclosure pertains. Although any methods and materials similar to or equivalent to those described herein can be used in the practice or testing of the devices and methods, the preferred methods and materials are described herein.

Moreover, reference to an element by the indefinite article "a" or "an" does not exclude the possibility that more than one element is present, unless the context clearly requires that there be one and only one element. The indefinite article "a" or "an" thus usually means "at least one." Likewise, the terms "have," "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. For example, the expressions "A has B," "A comprises B" and "A includes B" may refer both to a situation in which, besides B, no other element is present in A (*i.e.,* a situation in which A solely and exclusively consists of B) or to a situation in which, besides B, one or more further elements are present in A, such as element C, elements C and D, or even further elements.

### Overview

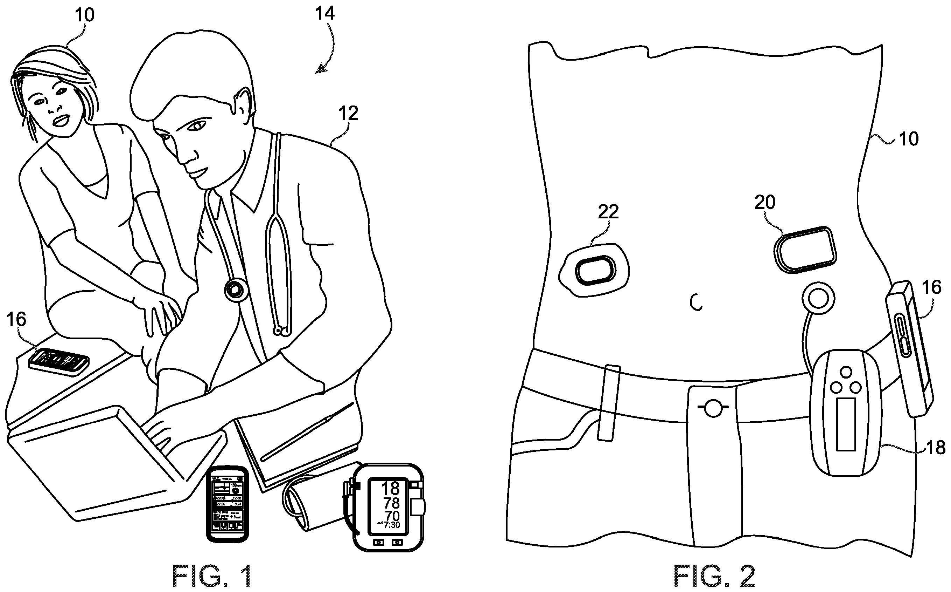

Fig. 1 shows a person with diabetes 10 and a health care provider 12 in a clinical environment 14. A person with diabetes 10 can use a handheld analyte meter such as a blood glucose meter or a combination blood glucose meter and infusion pump controller 16 as part of the person's therapy. A person with Type 1 diabetes can perform blood glucose measurements seven (7) or more times a day, and the need to perform blood glucose measurement can be urgent for events such as a feeling of low blood glucose, hypoglycemia, and prior to eating for calculating an insulin bolus. The health care provider 12 can manage the person with diabetes' 10 diagnostic and therapy information using diabetes management programs such as Accu-Chek^{®} 360 Diabetes Management System software and Accu-Chek^{®} Pump Configuration software operating on a computer, accessed via the World Wide Web, or both.

Fig. 2 shows a person with diabetes 10 wearing a variety of diabetes devices. The person with diabetes 10 in a self-care environment can use a variety of diabetes devices such as an ambulatory infusion pump 18, a patch infusion pump 20 that adheres to the person's skin, and a continuous glucose monitor 22. These diabetes devices are intended to be used by the person with diabetes 10 during daily activities such as outside under variable seasonal temperatures.

Alternatively, a handheld blood glucose meter or a combination handheld blood glucose meter and infusion pump controller can be used by persons with diabetes to measure blood glucose. As shown in Fig, 3, a test element 24 (or biosensor) can be inserted into the meter 16, where the test element 24 has a collection area 26 on one end extending away from the meter 16. A small drop of blood is placed on the collection area 26 to initiate an enzymatic reaction for electrochemical or photometric analysis to produce a blood glucose measurement. This enzymatic reaction is sensitive to temperature, so an acceptable temperature range is established for performing accurate blood glucose measurements, such as from about 4°C to about 45°C (about 39°F to about 113°F), from about 6°C to about 44°C (about 43°F to about 111°F); from about 4°C to about 40°C (about 39°F to about 104°F); or from about 10°C to about 45°C (about 50°F to about 113°F) and the like. Alternatively, the acceptable enzymatic reaction temperature range can be from about 5°C to about 40°C, from about 10°C to about 35°C, from about 15°C to about 30°C, or from about 20°C to about 25°C. Alternatively still, the acceptable enzymatic reaction temperature range can be from about 5°C to about 10°C, from about 10°C to about 15°C, from about 15°C to about 20°C, from about 20°C to about 25°C, from about 25°C to about 30°C, from about 30°C to about 35°C, from about 35°C to about 40°C, from about 40°C to about 45°C, or about 45°C. Also, the acceptable enzymatic reaction temperature can be about 5°C, about 10°C, about 15°C, about 20°C, about 25°C, about 30°C, about 35°C, about 40°C or about 45°C.

As used herein "about" means within a statistically meaningful range of a value or values such as, for example, a stated concentration, length, width, height, angle, weight, molecular weight, pH, sequence identity, time frame, temperature or volume. Such a value or range can be within an order of magnitude, typically within 20%, more typically within 10%, and even more typically within 5% of a given value or range. The allowable variation encompassed by "about" will depend upon the particular system under study, and can be readily appreciated by one of skill in the art.

If the enzymatic reaction occurs outside the acceptable temperature range, the test element will be operating outside of specifications and could produce an inaccurate blood glucose measurement. Prior to beginning a blood glucose measurement, a temperature sensor located inside the meter housing measures temperature to determine whether the temperature is within the acceptable temperature range. If the temperature is within the acceptable temperature range, a glucose measurement is performed, and, if the temperature is outside the acceptable temperature range, a glucose measurement is not performed.

As handheld analyte meters, such as blood glucose meters and combination blood glucose meters and infusion pump controllers, have been enhanced with features such as color displays, multiple microprocessors, and wireless communications power consumption has increased. Increased power consumption also produces internally generated heat that can adversely affect blood glucose measurements. An example of power management to control internal heating in a combination pump controller and blood glucose meter is disclosed in US Patent Application Publication No. 2012/0095312.

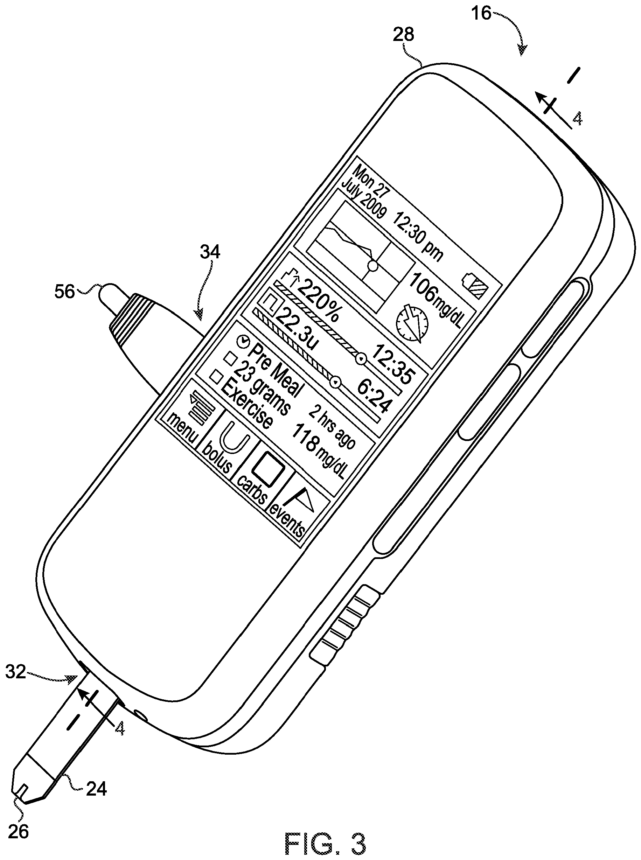

Increased power consumption also has increased battery energy demands leading to the use of rechargeable batteries. As also shown in Fig. 3, recharging can be performed by connecting a cable 56 to the meter 16 that supplies power for recharging or by placing the meter 16 configured for inductive charging on a charging pad. During recharging, the charging current and electrochemical reactions cause the battery to self-heat, which causes a temperature rise inside the housing. Also during recharging, the person with diabetes 10 can have an urgent need to perform a blood glucose test. If the meter 16 is recharging by a cable, the person with diabetes 10 would be required to disconnect the cable 56 from the meter 16 for safety to enable the blood glucose measurement. During this interruption of charging for an urgent blood glucose measurement, battery self-heating can pose the greatest risk for interfering with the blood glucose measurement. When the blood glucose meter 16 rises in temperature, the meter's 16 estimate for the acceptable temperate range to perform a blood glucose test can be inaccurate. An inaccurate temperature measurement can prevent a blood glucose measurement from being performed when the collection area 26 is actually within an acceptable temperature range (*e.g.,* a high lock-out condition caused by battery self-heating). An inaccurate temperature measurement also can permit a blood glucose measurement to be performed when the test element's sample collection area is outside the acceptable temperature range (e.g., masking a low lock-out condition caused by battery self-heating).

The devices and methods described herein therefore are useful in a variety of applications and address the problems noted above with self-heating that may result from charging and/or using a rechargeable battery. For example, rechargeable, handheld devices are routinely used to assay a variety of analytes for diagnosing and monitoring disease and treatment. Examples of such useful analytes include, but are not limited to, carbohydrates such as glucose, ketones such as beta-hydroxybutyrate, and lipids such as triglycerides. Of particular interest herein are analytes related to diagnosing and monitoring diabetes, especially assaying an analyte such as glucose.

### Devices

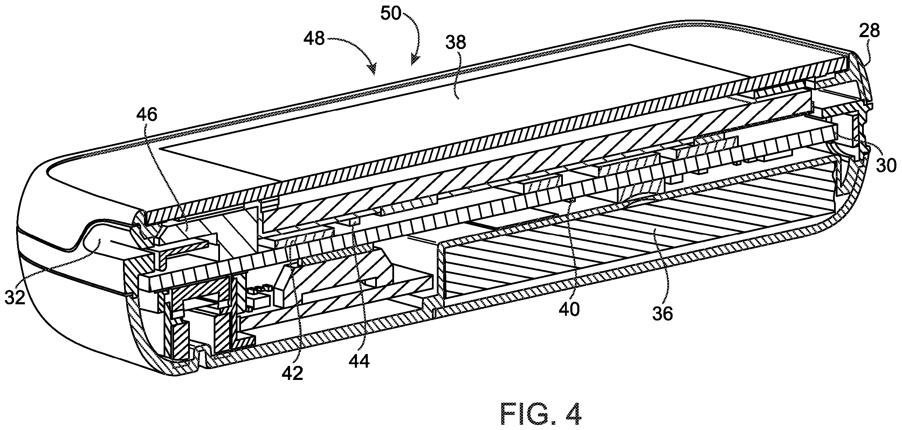

Fig. 3 shows a perspective view of an exemplary handheld analyte meter 16 with a rechargeable battery 36, and Fig. 4 shows a perspective cut-away view of the exemplary handheld analyte meter 16 with a rechargeable battery 36. The handheld analyte meter 16 with recharging control for controlling self-heating to improve analyte testing includes a device housing 28, a cable connector 34, a rechargeable battery 36, a display 38, a first temperature sensor 40, a measurement module 42, a second temperature sensor 44, a test element connector 46, a battery charger 48, and a charging control 50.

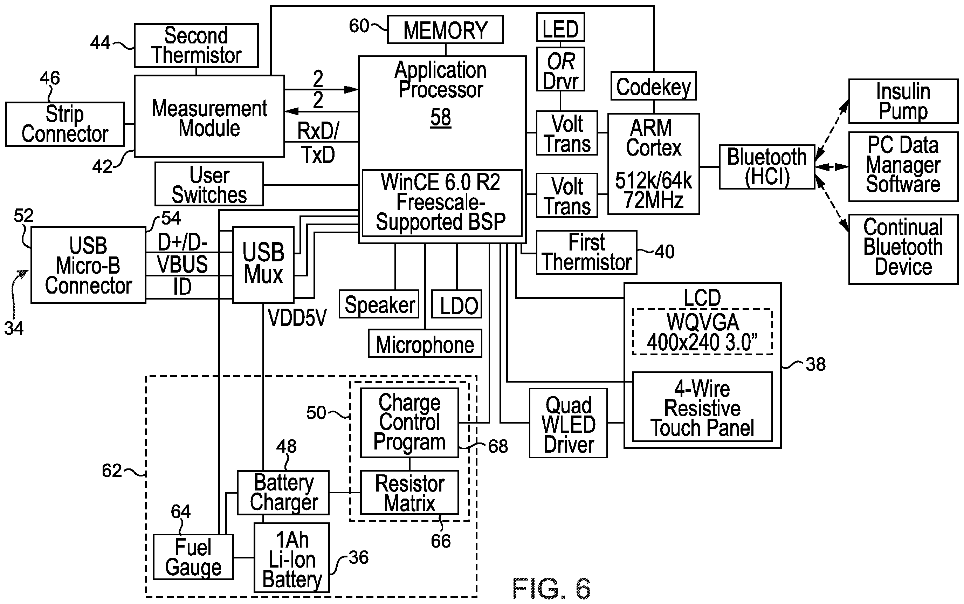

The housing 28 is formed from a thermoplastic that both encloses handheld analyte meter 16 components and serves as a frame for carrying components. The housing 28 is substantially sealed except for the test element port 32 that is open to receive a test element 24, where some airflow can occur through the test element port 32 to the housing interior. Fig. 6 shows that the cable connector 34 is carried in the housing 28 with a first end 52 exposed outside the housing for connecting to a charging source and a second end 54 coupled the circuit board 30. The cable connector 34 is used for both communicating and recharging and can be a Universal Serial Bus (USB) mini connector and the like. When a cable 56 is attached to the cable connector 34 for recharging, the battery charger 48 can determine both the type and capacity of the power source for recharging. The general types of power sources available are a dedicated power source and a USB power source such as can be found on a computer USB port. Both the dedicated power source and auxiliary power source for USB have an available charging current of 500 mA; however, the auxiliary power source often limits charging current to below 500 mA due to constraints such as other power requirements.

The rechargeable battery 36 is carried inside the housing 28 and is coupled to the circuit board 30 and to the cable connector second end 54. During recharging the rechargeable battery 36 self-heats due to electrochemical reactions associated with recharging along with electrical impedance associated with the charging current. Heat rise generated by the rechargeable battery during recharging ranges from about 2°C to about 4°C (about 3.6°F to about 7.2°F) depending upon the charging current. The rechargeable battery 36 can be a lithium ion battery rated at 3.7 VDC and 1150 mAh and has a predetermined charge level in the range from about 300 mA to about 1150 mA such as, for example, less than about 450 mA.

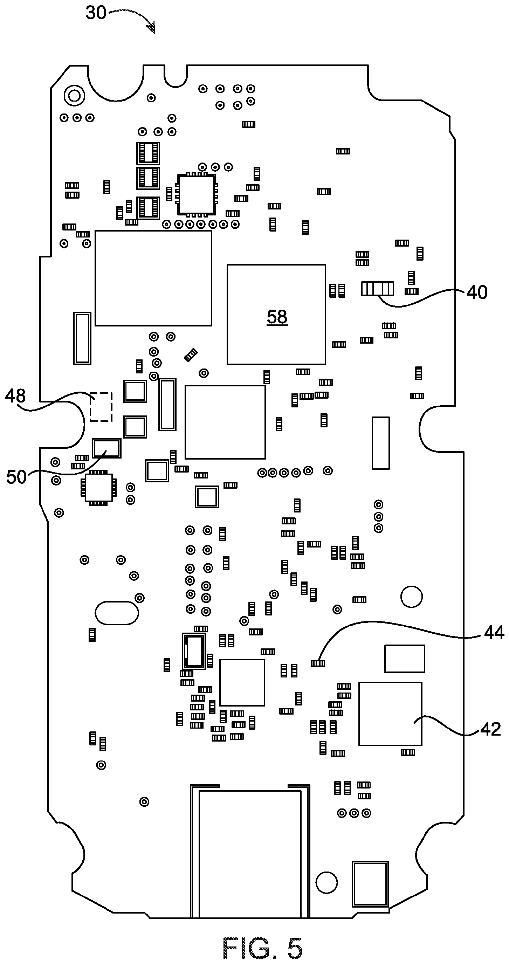

Fig. 5 shows a printed circuit board 30 for a handheld analyte meter 16 with a rechargeable battery 36. The spatial relationship is shown among the main processor 58, first temperature sensor 40, measurement module 42, and second temperature sensor 44. Additionally shown are the battery charger 48 and charging control 50.

Fig. 6 shows a block diagram of a handheld analyte meter 16 with a rechargeable battery 36. The handheld analyte meter 16 includes a housing (not shown), a cable connector 34, a rechargeable battery 36, a main processor 58 with memory 60, a display 38, a first temperature sensor 40, a measurement module 42, a second temperature sensor 44, a test element connector 46, and a charging module 62 including a battery charger 48 and a charging control 50. The main processor 58, also known as the user interface processor, can be a Freescale i.MX233 application processor using an operating system such as Microsoft^{®} WinCE. Memory 60 coupled to the process can be both volatile Synchronous Dynamic Random Access Memory (SDRAM) and non-volatile flash memory. The display 38 can be a liquid crystal display (LCD) having a resistive touch panel overlaid that cooperates with the display to form user interface buttons.

The first temperature sensor 40 can be a thermistor and is carried on the circuit board 30 near the main processor 58 and rechargeable battery 36. The first temperature sensor 40 is used for measuring a first temperature inside the housing 28 prior to beginning a charging session that is compared to a first temperature range. The first temperature range is selected to balance self-heating against charging time such as, for example, in a range from about 18°C to about 27°C (about 64°F to about 81°F). Alternatively, the first temperature range can be from about 19°C to about 26°C, from about 20°C to about 25°C, from about 21°C to about 24°C, or from about 22°C to about 23°C. Also, the first temperature can be about 18°C, about 19°C, about 20°C, about 21°C, about 22°C, about 23°C, about 24°C, about 25°C, about 26°C, or about 27°C.

The measurement module 42, sometimes referred to as a measurement engine, is carried on the circuit board 30 near the test element port 32 and coupled to the main processor 58, the second temperature sensor 44, and a test element connector 46. The measurement module 42 can be an Application Specific Integrated Circuit (ASIC) with a module processor and module memory that contains measurement firmware that perform the electrochemistry or photometric analysis of the test element 24 once a blood sample has been applied to produce a blood glucose measurement. The measurement module 42 also directly controls the second temperature sensor 44.

The measurement module 42 determines high lock-out and low lock-out conditions to prevent blood glucose measurements based upon the second temperature of the second temperature sensor 44. The measurement module 42 determines a high lock-out condition when the temperature as measured by the second temperature sensor 44 is higher than the specific range for the enzymatic reaction such as above about 45°C (113°F), above about 44°C (about 111°F), above about 43°C (109°F), above about 42°C (108°F), above about 41°C (106°F), or even above about 40°C (about 104°F). Likewise, the measurement module 42 determines a low lock-out condition when the temperature measured by the second temperature sensor 44 is lower than the specific range for the enzymatic reaction such as lower than about 4°C (39°F), lower than about 5°C (41°F), lower than about 6°C (about 43°F), lower than about 7°C (45°F), lower than about 8°C (46°F), lower than about 9°C (48°F), or even lower than about 10°C (about 50°F).

The temperature range between the low lock-out and high lock-out is the acceptable temperature range as measured by the second temperature sensor 44 for performing blood glucose measurements, which is from, for example, about 4°C to about 45°C (about 39°F to about 113°F), from about 6°C to about 44°C (about 43°F to about 111°F), from about 4°C to about 40°C (about 39°F to about 104°F), from about 10°C to about 45°C (about 50°F to about 113 °F) and the like.

During recharging, battery self-heating can potentially create a false high-lock out condition to prevent performing a blood glucose measurement when the test element is actually within the acceptable temperature range to perform a blood glucose measurement. Also during recharging, battery self-heating can potentially mask a low lock-out condition that would permit performing a blood glucose measurement when the actual test element temperature is lower than the acceptable temperature range to perform a blood glucose measurement. If a low lock-out condition is masked by battery 36 self-heating, a person with diabetes 10 could perform a blood glucose measurement outside of the specifications for the test element 24. This blood glucose measurement performed outside of test element 24 specifications could then be used for a therapy adjustment such as delivering an insulin bolus with unpredictable results.

The second temperature sensor 44 can be a thermistor carried on the circuit board 30 near the measurement module 42 for measuring a second temperature inside the housing 28. By locating the second temperature sensor 44 near the measurement module 42, the second temperature sensor 44 more closely reflects the test element temperature than the first temperature sensor 40 that more closely reflects the battery temperature.

The measurement module 42 controls the second temperature sensor 44. Prior to beginning an analyte test, the measurement module 42 obtains a second temperature from the second temperature sensor 44, and the second temperature is compared to a second temperature range. The second temperature range is the test element-specified temperature operating range such as, for example, from about 6°C to about 44°C (about 43°F to about 111°F). The measurement module 42 compares the second temperature to the second temperature range to determine whether to impose a high lock-out or low-lock out to prevent a blood glucose measurement from being conducted because the test element temperature could be outside its specified temperature range.

The test element connector 46 is carried on the circuit board 30 near the test element port 32. The test element connector 46 is coupled to the measurement module 42 and is configured to receive a test element 24. When the test element 24 is inserted through the test element port 32 into the test element connector 46, test element 24 contacts form an electrical connection with test element connector 46 terminals and to the measurement module 42. There is little thermal conduction between the test element 24 and the test element connector 46 because the test element 24 substrate is plastic. The charging module 62 is discussed below.

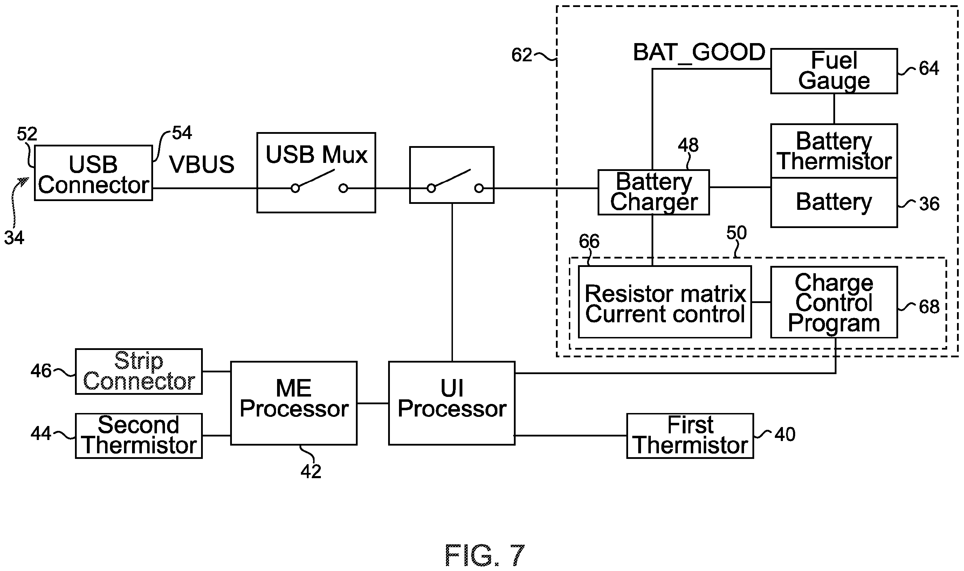

Fig. 7 shows a block diagram of a charging module 62 including a battery charger 48, a charging control 50, a fuel gauge 64, and a rechargeable battery 36. The battery charger 48 is carried on the circuit board 30 and is coupled to the cable connector 34 that charges the rechargeable battery 36 up to a maximum charging current until the rechargeable battery 36 reaches a predetermined charge level. The battery charger 48 also is coupled to fuel gauge 64. The battery charger 48 receives 5 VDC from the USB port and converts it to 4.2 VDC for charging the battery 36, and the charging current also is controlled by the battery charger 48. The fuel gauge 64 provides a battery 36 temperature lock-out for charging to prevent damage to the battery. The battery 36 temperature charging acceptance range is wide enough, so it does not interact with the high lock-out and low lock-out.

The charging control 50 includes a charging resistor matrix 66 and a charge control program 68. The charging resistor matrix 66 is used to change the control voltage of the battery charger 48 to limit charging current. The charging control 50 is carried on the circuit board 30 and is coupled to the battery charger 48 that prior to initiating a charging session selects the maximum charging current that does not change during the charging session. The maximum charging current is selected based upon capacity of a charging source, and the first temperature compared to the first temperature range. The maximum charging current can be in a range from about 50 mA to about 450 mA, from about 75 mA to about 425 mA, from about 100 mA to about 400 mA, from about 125 mA to about 375 mA, from about 150 mA to about 350 mA, from about 175 mA to about 325 mA, from about 200 mA to about 300 mA, from about 225 mA to about 275 mA or about 250 mA. Alternatively, the range can be from about 50 mA to about 100 mA, from about 100 mA to about 150 mA, from about 150 mA to about 200 mA, from about 200 mA to about 250 mA, from about 250 mA to about 300 mA, from about 300 mA to about 350 mA, from about 350 mA to about 400 mA, or from about 400 mA to about 450 mA. Also, the maximum charging current can be about 50 mA, about 100 mA, about 150 mA, about 200 mA, about 220 mA, about 250mA, about 300 mA, about 350 mA, about 360 mA, about 370mA, about 380 mA, about 390 mA, about 400 mA, about 410 mA, about 420 mA, about 430 mA, about 440 mA, and about 450 mA.

### Methods

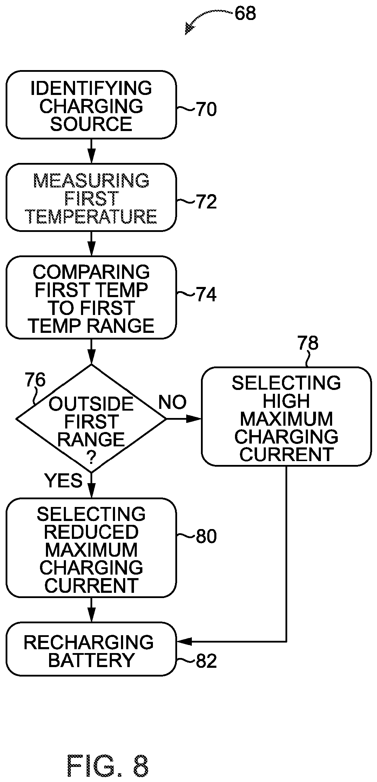

Fig. 8 shows a flowchart of a charge control program 68 for selecting a charge current for the battery charger. The charge control program 68 is stored in memory 60 and is executed by the main processor 58. The charge control program 68 includes the steps of identifying the charging source 70, measuring a first temperature 72, comparing the first temperature to a first temperature range 74, determining whether the first temperature is outside the first temperature range 76, selecting high maximum charging current if the first temperature is not outside the first temperature range 78 or selecting a low/reduced maximum charging current if the first temperature is outside the first temperature range 80, and charging the battery 82.

When a charging source is plugged into the cable connector 52, the battery charger 48 determines whether the charging source is a dedicated charger or a charging source from a device such as a computer. Dedicated charging sources typically have a capacity of at least about 500 mA. Device charging sources such as a computer typically have a capacity from about 100 mA to about 500 mA. The first temperature is read to determine the temperature inside the housing. This first temperature is compared against a first temperature range that is selected to balance self-heating against charging time such as in the range from about 18°C to about 27°C (about 64°F to about 81°F). If the first temperature is within the first temperature range, the charge control program will select a highest maximum charging current of 500 mA or less such as 380 mA. If the first temperature is outside the first temperature range, the charge control program will select a reduced maximum charging current that is under 380 mA, such as 300 mA, 220 mA, or 50 mA.

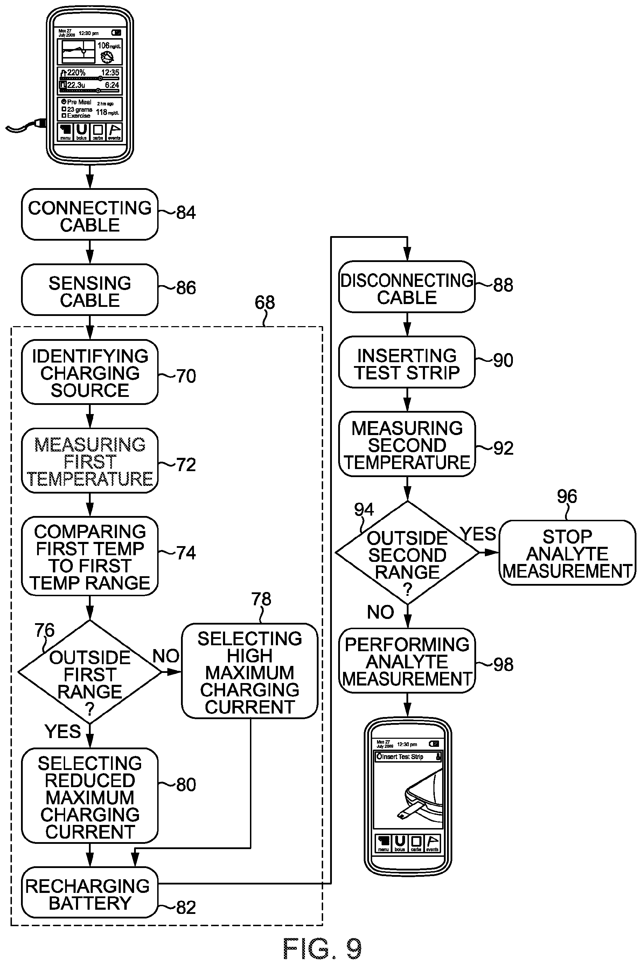

Fig. 9 shows a flowchart of a method for recharging a handheld analyte meter 16 with a rechargeable battery 36. The method includes the steps of connecting a cable 84, sensing the cable 86, identifying the charging source 70, measuring a first temperature 72, determining whether the first temperature was outside a first temperature range 76, selecting a high maximum charging current if the first temperature is not outside the first temperature range 78 or selecting a reduced maximum charging current if the first temperature is outside the first temperature range 80, recharging a rechargeable battery 82, disconnecting the cable 88, inserting a test element 90, measuring a second temperature 92, determining whether the second temperature is within a second temperature range 94, stopping analyte measurement 96 if the second temperature is outside the second temperature range or performing the analyte test 98 if the second temperature is within the second temperature range.

To prepare for recharging, the user connects a cable 84 that is coupled to a charging source to a cable connector 52 on the handheld analyte meter 16, and the user turns the analyte meter 16 "on" if it was "off." The cable 56 provides both power and communications such as a USB cable. The analyte meter 16 senses 86 when the cable 56 is connected to the cable connector 52 and disables the analyte meter 16 from conducting an analyte test for electrical safety. The analyte meter 16 also identifies the type of charging source 70 and the charging source capacity.

Prior to initiating the recharging session, the analyte meter 16 measures a first temperature in the analyte meter 16 with a first temperature sensor 40 located inside the analyte meter housing 28 near the rechargeable battery 36. The analyte meter 16 determines whether the first temperature is outside a first temperature range to reduce the maximum charging current. The first temperature range is selected to balance self-heating against charging time such as, for example, in the range from about 18°C to about 27°C (about 64°F to about 81°F).

A maximum charging current is selected prior to beginning a charging session that does not change during the charging session. The maximum charging current is selected based upon capacity of a charging source and the first temperature compared to the first temperature range in the range from, for example, about 50 mA to about 380 mA or even one of the discrete values of 50 mA, 220 mA, 300 mA, 380 mA, and 450 mA. Although the maximum charging current is selected, the actual charging current can be zero because the fuel gauge 64 provides a battery temperature lock-out to prevent damage to the battery 36. If the battery temperature is too high, the charging module 62 will disable charging to avoid damaging the battery 36. The maximum charging current does not change during the charging session because the maximum charging current is selected to avoid self-heating that would create a high lock-out condition or mask a low-lock out condition.

If the first temperature is within the first temperature range, the charge control program 50 will select a high maximum charging current of 500 mA or less such as 380 mA. If the first temperature is outside the first temperature range, the charge control program 50 will select a reduced maximum charging current that is under 380 mA, such as 300 mA, 220 mA, or 50 mA. After the maximum charging current is selected, the recharging session begins by recharging the rechargeable battery 36 of the analyte meter 16 at up to the maximum charging current selected.

When the recharging session is completed or if the user desires to interrupt the recharging session to perform an analyte test, such as an urgent blood glucose measurement due to feelings of hypoglycemia or to calculate an insulin bolus. The user disconnects the cable 56 from the analyte meter 16. The analyte meter 16 is now enabled to perform an analyte test such as a blood glucose measurement. The user inserts a test element 24 into a test element port 32 on the analyte meter 16, and the measurement module 42 is activated to measure the second temperature with the second temperature 44 sensor located inside the analyte meter housing 28 near the measurement module 42. The measurement module 42 determines whether the second temperature measurement is within a second temperature range to conduct an analyte test. The analyte meter 16 then performs the analyte test if the second temperature is within the second temperature range. The analyte meter will report an error if the second temperature is outside the second temperature range.

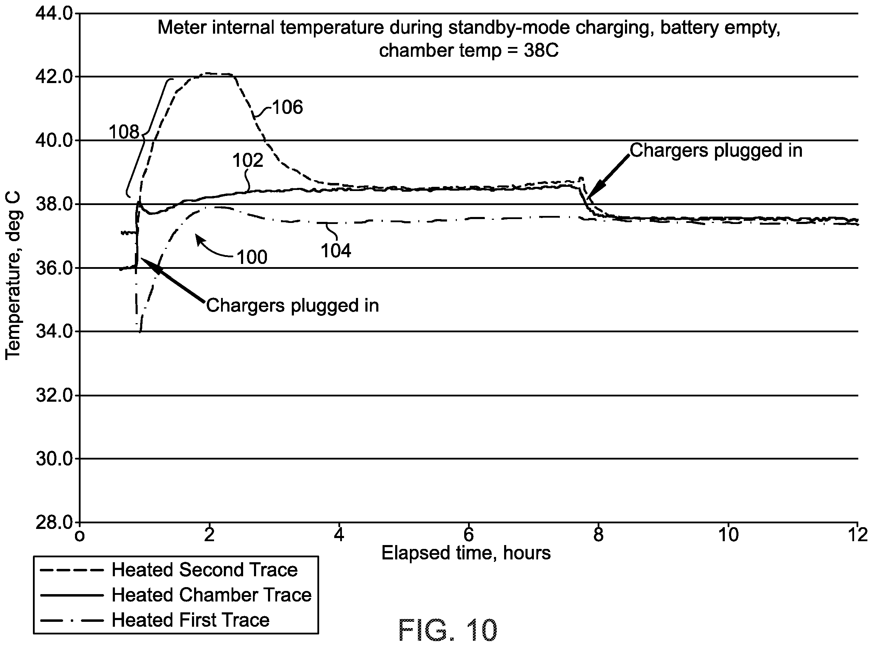

Fig. 10 shows test results in a heated environmental chamber comparing temperature increases during recharging for a handheld analyte meter with charging control (first analyte meter) and a handheld analyte meter without charging control (second analyte meter). The environment chamber was heated to 38°C (100°F), and the first analyte meter and the second analyte meter were placed in the environmental chamber. To measure temperatures that could cause the measurement module 42 to impose a high lock-out condition or a low lock-out condition, a thermocouple was attached by a thermally conductive adhesive to the second temperature sensor of each of the first analyte meter and the second analyte meter. A third thermocouple was placed in the environmental chamber to measure internal chamber temperature as shown by the heated chamber trace.

The testing procedure began with opening environment chamber door to plug in a USB cable with a dedicated charger to each meter's cable connector. Opening the environmental chamber door caused a temporary transient temperature decrease of about 4°C (7.2°F) as shown by the heated chamber trace. The first analyte meter is shown in the test results as a heated first trace, and the second analyte meter is shown in the test results as a heated second trace. As the meters are allowed to charge over several hours, the temperature characteristics were observed. The second analyte meter without the charging control program charged at 380 mA and experienced an initial temperature rise of about 4°C compared to the first analyte meter with the charging control program 50. The initial temperature rise on the second analyte meter without the charge control program would have caused a high lock-out condition, and incorrectly prevented the second analyte meter because the heated chamber trace shows the test element temperature would have been within an acceptable temperature range to perform a blood glucose test.

In contrast, the first analyte meter with the charge control program charged at 220 mA, and the heated first trace shows that the first analyte meter did not enter a high lock-out condition during the entire test.

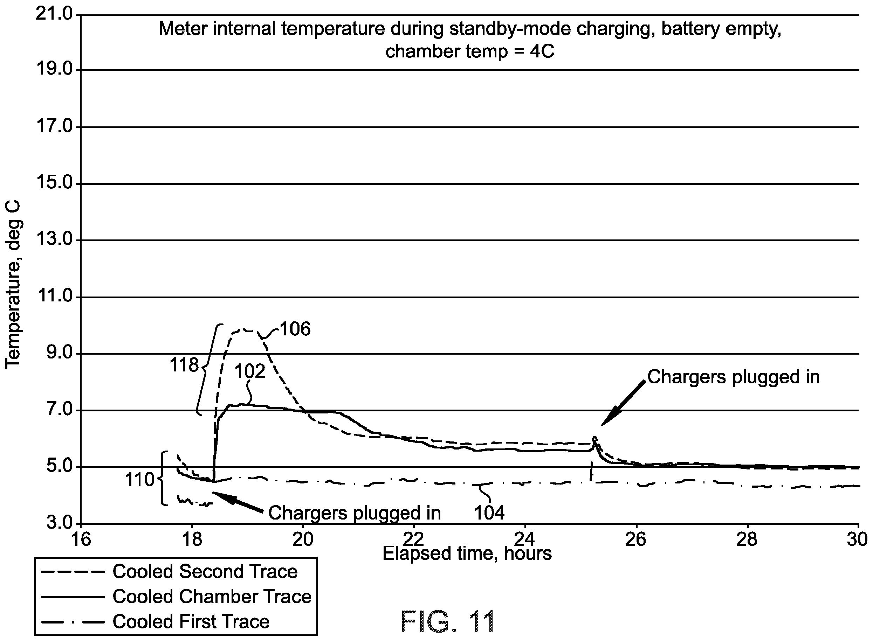

Fig. 11 shows test results in a cooled environmental chamber comparing temperature increases during recharging for a handheld analyte meter with charging control (*i.e.,* first analyte meter) and a handheld analyte meter without charging control (second analyte meter). The environment chamber was cooled to 4°C (39.2°F), and the first analyte meter and the second analyte meters were placed in the environmental chamber. To measure temperatures that could cause the measurement module 42 to impose a high lock-out condition or a low lock-out condition, a thermocouple was attached by a thermally conductive adhesive to the second temperature sensor of the first analyte meter and the second analyte meter. A third thermocouple was placed in the environmental chamber to measure interval chamber temperature as shown by the cooled chamber trace.

The testing procedure began with opening environment chamber door to plug in a USB cable with a dedicated charger to each meter's cable connector. Opening the environmental chamber door caused a temporary transient temperature increase of about 3°C (5.4°F) as shown by the cooled chamber trace. The first analyte meter is shown in the test results as a cooled first trace, and the second analyte meter is shown in the test results as a cooled second trace. As the meters are allowed to charge over several hours, their temperature characteristics were observed. The second analyte meter without the charge control program charged at 380 mA and experienced an initial temperature rise of about 4°C (10.8°F) compared to the first analyte meter with the charge control program. The initial temperature rise on the second analyte meter would have masked a low lock-out condition, and incorrectly allowed a blood glucose test to be performed despite the strip temperature being too low as shown by the cooled chamber trace. In contrast, the first analyte meter with the charge control program charged at 220 mA and did not mask a low lock-out condition during the entire test.

### Listing of Reference Numbers

10 person with diabetes
12 health care provider
14 clinical environment
16 handheld analyte meter
18 ambulatory infusion pump
20 patch infusion pump
22 continuous glucose monitor
24 test element/test strip
26 collection area
28 device housing
30 circuit board
32 test element port/test strip port
34 cable connector
36 rechargeable battery
38 display
40 first temperature sensor
42 measurement module
44 second temperature sensor
46 test element connector/test strip connector
48 battery charger
50 charging control
52 first end of cable connector
54 second end of cable connector
56 connecting cable
58 main processor
60 memory
62 charging module
64 fuel gauge
66 charging resistor matrix
68 charge control program
70 identifying a charging source
72 measuring a first temperature
74 comparing the first temperature to a first temperature range
76 determining whether the first temperature is outside first temperature range
78 selecting a high maximum charging current if the first temperature is not outside the first temperature range
80 selecting a low maximum charging current if the first temperature is outside the first temperature range
82 charging a battery
84 connecting a cable
86 sensing the cable
88 disconnecting the cable
90 inserting a test element
92 measuring a second temperature
94 determining whether the second temperature is within a second temperature range
96 stopping analyte measurement if the second temperature is outside the second temperature range
98 performing analyte measurement if the second temperature is within the second temperature range

## Claims

1. A handheld analyte meter (16) with recharging control for regulating self-heating to improve analyte testing, the meter comprising:
a housing (28) having a circuit board (30) carried inside the housing and a test element port (32);
a cable connector (34) carried in the housing with a first end exposed outside the housing for connection to a charging source and a second end coupled to the circuit board;
a rechargeable battery (36) carried inside the housing and coupled to the circuit board and coupled to the cable connector second end;
a main processor (58) carried on the circuit board and coupled to the rechargeable battery, the main processor having memory;
a display (38) coupled to the circuit board and the main processor;
a first temperature sensor (40) carried on the circuit board near the main processor and
rechargeable battery for measuring a first temperature inside the housing prior to beginning a charging session that is compared to a first temperature range;
a measurement module (42) carried on the circuit board near the test element port and
coupled to the main processor;
a second temperature sensor (44) carried on the circuit board near the measurement module for measuring a second temperature inside the housing prior to beginning an analyte test that is compared to a second temperature range;
a test element connector (46) carried on the circuit board near the test element port. the test element connector coupled to the measurement module and configured to receive a test element;
a battery charger (48) carried on the circuit board and coupled to the cable connector that charges the rechargeable battery up to a maximum charging current until the rechargeable battery reaches a predetermined charge level; and
a charging control (50) carried on the circuit board and coupled to the battery charger that prior to the beginning of a charging session selects the maximum charging current that does not change during the charging session,
wherein if the first temperature is within the first temperature range, the charging control selects a high maximum charging current for the maximum charging current, and
wherein if the first temperature is outside the first temperature range, the charging control selects a reduced maximum charging current for the maximum charging current, wherein if the first temperature is lower than the first temperature range, the maximum charging current is reduced to lower the risk of masking a low lock-out that permits an analyte test when the second temperature is higher than a lowest value of the second temperature range.

2. The handheld analyte meter of Claim 1, wherein if the first temperature is higher than the first temperature range, the maximum charging current is reduced to lower the risk of a high lock-out that prevents an analyte test when the second temperature is higher than the second temperature range.

3. The handheld analyte meter of Claim 2, wherein the charging current is in a range from about 50 mA to about 450 mA.

4. The handheld analyte meter of Claim 1, wherein the test element has a test element temperature range from about 4°C to about 45°C.

5. The handheld analyte meter of Claim 1, wherein the handheld rechargeable analyte meter is a handheld infusion pump controller with blood glucose meter or a self-testing blood glucose meter.

6. A method of controlling handheld analyte meter recharging to regulate self-heating for improved analyte testing, the method comprising the steps of:
connecting a cable coupled to a charging source to a cable connector on the handheld analyte meter and turning the analyte meter on if the analyte meter was off when the cable is connected to the cable connector;
sensing the cable is connected to the cable connector and disabling the analyte meter from conducting an analyte test for safety;
identifying the charging source capacity by the analyte meter;
measuring a first temperature in the analyte meter with a first temperature sensor located inside the analyte meter housing near the rechargeable battery prior to the beginning of a charging session;
determining whether the first temperature is within a first temperature range;
selecting a high maximum charging current for a maximum charging current prior to the beginning of a charging session that does not change during the charging session based upon capacity of a charging source and if the first temperature is within the first temperature range;
selecting a reduced maximum charging current for the maximum charging current prior to the beginning of the charging session that does not change during the charging session based upon capacity of the charging source and if the first temperature is outside the first temperature range;
recharging a rechargeable battery of the analyte meter at up to the maximum charging current selected;
disconnecting the cable from the analyte meter and enabling the analyte meter to perform an analyte test;
inserting a test element into a test element port on the analyte meter;
measuring a second temperature with a second temperature sensor located inside the analyte meter housing near a measurement engine;
determining whether the second temperature measurement is within a second temperature range to conduct an analyte test; and
performing the analyte test if the second temperature is within the second
temperature range,
wherein if the first temperature is lower than the first temperature range maximum charging current is reduced to lower a risk of masking a low lock- out that permits an analyte test when the second temperature is higher than a lowest value of the second temperature range.

7. The method of Claim 6, wherein if the first temperature is higher than the first temperature range the maximum charging current is reduced to lower a risk of a high lock-out that prevents an analyte test when the second temperature is higher than the second temperature range.

8. The method of Claim 6, wherein the charging current is selected in the range from about 50 mA to about 450 mA.

9. The method of Claim 6, wherein the test element has a temperature range from about 4°C to about 45°C.

10. The method of Claim 6 wherein the handheld analyte meter is selected from one of a handheld infusion pump controller with blood glucose meter and a handheld blood glucose meter.

## Patentansprüche

1. Tragbares Analytmessgerät (16) mit Wiederaufladungssteuerung zum Regeln des Selbsterhitzens zur Verbesserung des Analyttestens, wobei das Messgerät Folgendes umfasst:
ein Gehäuse (28) mit einer Leiterplatte (30), die in dem Gehäuse getragen wird, und einer Testelementöffnung (32);
einen in dem Gehäuse getragenen Kabelverbinder (34), von dem ein erstes Ende außerhalb des Gehäuses zur Verbindung mit einer Ladungsquelle freiliegt und ein zweites Ende an die Leiterplatte gekoppelt ist;
eine wiederaufladbare Batterie (36), die in dem Gehäuse getragen wird und an die Leiterplatte gekoppelt ist und an das zweite Ende des Kabelverbinders gekoppelt ist;
einen Hauptprozessor (58), der auf der Leiterplatte getragen wird und an die wiederaufladbare Batterie gekoppelt ist, wobei der Hauptprozessor einen Speicher aufweist;
eine Anzeige (38), die an die Leiterplatte und den Hauptprozessor gekoppelt ist;
einen ersten Temperatursensor (40), der auf der Leiterplatte nahe dem Hauptprozessor und der wiederaufladbaren Batterie getragen wird, zum Messen einer ersten Temperatur in dem Gehäuse, bevor mit einem Ladungsvorgang begonnen wird, die mit einem ersten Temperaturbereich verglichen wird,
ein Messmodul (42), das auf der Leiterplatte nahe der Testelementöffnung getragen wird und an den Hauptprozessor gekoppelt ist;
einen zweiten Temperatursensor (44), der auf der Leiterplatte nahe dem Messmodul getragen wird, zum Messen einer zweiten Temperatur in dem Gehäuse, bevor mit einem Analyttest begonnen wird, die mit einem zweiten Temperaturbereich verglichen wird,
einen Testelementverbinder (46), der auf der Leiterplatte nahe der Testelementöffnung getragen wird, wobei der Testelementverbinder an das Messmodul gekoppelt und zum Aufnehmen eines Testelements ausgelegt ist;
ein Batterieladegerät (48), das auf der Leiterplatte getragen wird und an den Kabelverbinder gekoppelt ist und die wiederaufladbare Batterie bis zu einem maximalen Ladungsstrom lädt, bis die wiederaufladbare Batterie einen vorbestimmten Ladungsstand erreicht; und
eine Ladungssteuerung (50), die auf der Leiterplatte getragen wird und an das Batterieladegerät gekoppelt ist und vor Beginn eines Ladungsvorgangs den maximalen Ladungsstrom auswählt, der sich während des Ladungsvorgangs nicht ändert,
wobei, wenn die erste Temperatur innerhalb des ersten Temperaturbereiches liegt, die Ladungssteuerung einen hohen maximalen Ladungsstrom für den maximalen Ladungsstrom auswählt, und
wobei, wenn die erste Temperatur außerhalb des ersten Temperaturbereiches liegt, die Ladungssteuerung einen reduzierten maximalen Ladungsstrom für den maximalen Ladungsstrom auswählt,
wobei, wenn die erste Temperatur niedriger ist als der erste Temperaturbereich, der maximale Ladungsstrom reduziert wird, um das Risiko des Maskierens einer Schwachblockierung, die einen Analyttest ermöglicht, wenn die zweite Temperatur höher ist als ein niedrigster Wert des zweiten Temperaturbereiches, zu verringern.

2. Tragbares Analytmessgerät nach Anspruch 1, wobei, wenn die erste Temperatur höher ist als der erste Temperaturbereich, der maximale Ladungsstrom reduziert wird, um das Risiko einer Starkblockierung, die einen Analyttest verhindert, wenn die zweite Temperatur höher ist als der zweite Temperaturbereich, zu verringern.

3. Tragbares Analytmessgerät nach Anspruch 2, wobei der Ladungsstrom im Bereich von etwa 50 mA bis etwa 450 mA liegt.

4. Tragbares Analytmessgerät nach Anspruch 1, wobei das Testelement einen Testelementtemperaturbereich von etwa 4 °C bis etwa 45 °C aufweist.

5. Tragbares Analytmessgerät nach Anspruch 1, wobei das tragbare wiederaufladbare Analytmessgerät eine tragbare Infusionspumpensteuerung mit Blutglukosemessgerät oder ein Selbsttestblutglukosemessgerät ist.

6. Verfahren zum Steuern des Wiederaufladens eines tragbaren Analytmessgerätes zum Regeln des Selbsterhitzens zum besseren Analyttesten, wobei das Verfahren die folgenden Schritte umfasst:
Verbinden eines Kabels, das an eine Ladungsquelle gekoppelt ist, mit einem Kabelverbinder an dem tragbaren Analytmessgerät und Anschalten des Analytmessgerätes, wenn das Analytmessgerät aus ist, wenn das Kabel mit dem Kabelverbinder verbunden wird,
Erfassen, dass das Kabel mit dem Kabelverbinder verbunden ist, und zur Sicherheit Verhindern, dass das Analytmessgerät einen Analyttest durchführt;
Identifizieren der Ladungsquellenkapazität mittels des Analytmessgerätes;
Messen einer ersten Temperatur in dem Analytmessgerät mit einem ersten Temperatursensor, der sich in dem Analytmessgerätgehäuse nahe der wiederaufladbaren Batterie befindet, vor dem Beginn eines Ladungsvorgangs,
Bestimmen, ob die erste Temperatur innerhalb eines ersten Temperaturbereiches liegt,
Auswählen eines hohen maximalen Ladungsstroms für einen maximalen Ladungsstrom vor dem Beginn eines Ladungsvorgangs, der sich während des Ladungsvorgangs nicht ändert, basierend auf der Kapazität einer Ladungsquelle und wenn die erste Temperatur innerhalb des ersten Temperaturbereiches liegt,
Auswählen eines reduzierten maximalen Ladungsstroms für den maximalen Ladungsstrom vor dem Beginn eines Ladungsvorgangs, der sich während des Ladungsvorgangs nicht ändert, basierend auf der Kapazität einer Ladungsquelle und wenn die erste Temperatur außerhalb des ersten Temperaturbereiches liegt,
Wiederaufladen einer wiederaufladbaren Batterie des Analytmessgeräts bis zu dem ausgewählten maximalen Ladungsstrom;
Lösen des Kabels von dem Analytmessgerät und Zulassen, dass das Analytmessgerät einen Analyttest durchführt;
Einführen eines Testelements in eine Testelementöffnung an dem Analytmessgerät;
Messen einer zweiten Temperatur mit einem zweiten Temperatursensor, der sich in dem Analytmessgerätgehäuse nahe einer Messfunktionseinheit befindet,
Bestimmen, ob die zweite Temperaturmessung innerhalb eines zweiten Temperaturbereiches liegt, um einen Analyttest durchzuführen; und
Durchführen des Analyttests, wenn die zweite Temperatur innerhalb des zweiten Temperaturbereiches liegt,
wobei, wenn die erste Temperatur niedriger ist als der erste Temperaturbereich, der maximale Ladungsstrom reduziert wird, um das Risiko des Maskierens einer Schwachblockierung, die einen Analyttest ermöglicht, wenn die zweite Temperatur höher ist als ein niedrigster Wert des zweiten Temperaturbereiches, zu verringern.

7. Verfahren nach Anspruch 6, wobei, wenn die erste Temperatur höher ist als der erste Temperaturbereich, der maximale Ladungsstrom reduziert wird, um das Risiko einer Starkblockierung, die einen Analyttest verhindert, wenn die zweite Temperatur höher ist als der zweite Temperaturbereich, zu verringern.

8. Verfahren nach Anspruch 6, wobei der Ladungsstrom im Bereich von etwa 50 mA bis etwa 450 mA ausgewählt wird.

9. Verfahren nach Anspruch 6, wobei das Testelement einen Temperaturbereich von etwa 4 °C bis etwa 45 °C aufweist.

10. Verfahren nach Anspruch 6, wobei das tragbare Analytmessgerät aus einem von einer tragbaren Infusionspumpensteuerung mit Blutglukosemessgerät und einem tragbaren Blutglukosemessgerät ausgewählt wird.

## Revendications

1. Dispositif de mesure de substance à analyser portable (16) ayant une commande de recharge pour réguler l'autochauffage pour améliorer l'analyse de substance à analyser, le dispositif de mesure comprenant :
un boîtier (28) ayant une carte de circuit imprimé (30) transportée à l'intérieur du boîtier et un port d'élément d'analyse (32) ;
un connecteur de câble (34) transporté dans le boîtier ayant une première extrémité exposée à l'extérieur du boîtier pour la connexion à une source de charge et une seconde extrémité couplée à la carte de circuit imprimé ;
une batterie rechargeable (36) transportée à l'intérieur du boîtier et couplée à la carte de circuit imprimé et couplée à la seconde extrémité de connecteur de câble ;
un processeur principal (58) transporté sur la carte de circuit imprimé et couplé à la batterie rechargeable, le processeur principal ayant une mémoire ;
un dispositif d'affichage (38) couplé à la carte de circuit imprimé et au processeur principal ;
un premier capteur de température (40) transporté sur la carte de circuit imprimé à proximité du processeur principal et de la batterie rechargeable pour mesurer une première température à l'intérieur du boîtier avant le début d'une session de charge qui est comparée à une première plage de température ;
un module de mesure (42) transporté sur la carte de circuit imprimé à proximité du port d'élément d'analyse et couplé au processeur principal ;
un second capteur de température (44) transporté sur la carte de circuit imprimé à proximité du module de mesure pour mesurer une seconde température à l'intérieur du boîtier avant le début d'une analyse de substance à analyser qui est comparée à une seconde plage de température ;
un connecteur d'élément d'analyse (46) transporté sur la carte de circuit imprimé à proximité du port d'élément d'analyse, le connecteur d'élément d'analyse étant couplé au module de mesure et configuré pour recevoir un élément d'analyse ;
un chargeur de batterie (48) transporté sur la carte de circuit imprimé et couplé au connecteur de câble qui charge la batterie rechargeable jusqu'à un courant de charge maximal jusqu'à ce que la batterie rechargeable atteigne un niveau de charge prédéterminé ; et
une commande de charge (50) transportée sur la carte de circuit imprimé et couplée au chargeur de batterie qui avant le début d'une session de charge sélectionne le courant de charge maximal qui ne change pas pendant la session de charge,
dans lequel si la première température est dans la première plage de température, la commande de charge sélectionne un courant de charge maximal élevé pour le courant de charge maximal, et
dans lequel si la première température est en dehors de la première plage de température, la commande de charge sélectionne un courant de charge maximal réduit pour le courant de charge maximal, dans lequel si la première température est inférieure à la première plage de température, le courant de charge maximal est réduit pour diminuer le risque de masquage d'un faible verrouillage qui permet une analyse de substance à analyser lorsque la seconde température est supérieure à une valeur la plus faible de la seconde plage de température.

2. Dispositif de mesure de substance à analyser portable selon la revendication 1, dans lequel si la première température est supérieure à la première plage de température, le courant de charge maximal est réduit pour diminuer le risque d'un verrouillage élevé qui empêche une analyse de substance à analyser lorsque la seconde température est supérieure à la seconde plage de température.

3. Dispositif de mesure de substance à analyser portable selon la revendication 2, dans lequel le courant de charge est dans une plage allant d'environ 50 mA à environ 450 mA.

4. Dispositif de mesure de substance à analyser portable selon la revendication 1, dans lequel l'élément d'analyse a une plage de température d'élément d'analyse allant d'environ 4 °C à environ 45 °C.

5. Dispositif de mesure de substance à analyser portable selon la revendication 1, dans lequel le dispositif de mesure de substance à analyser rechargeable portable est un dispositif de commande de pompe à perfusion portable avec glucomètre ou un glucomètre d'auto-analyse.

6. Procédé de commande de recharge de dispositif de mesure de substance à analyser portable pour réguler l'autochauffage pour une analyse de substance à analyser améliorée, le procédé comprenant les étapes de :
connexion d'un câble couplé à une source de charge à un connecteur de câble sur le dispositif de mesure de substance à analyser portable et mise en marche du dispositif de mesure de substance à analyser si le dispositif de mesure de substance à analyser était éteint lorsque le câble est connecté au connecteur de câble ;
détection que le câble est connecté au connecteur de câble et désactivation du dispositif de mesure de substance l'empêchant de réaliser une analyse de substance à analyser pour des raisons de sécurité ;
identification de la capacité de source de charge par le dispositif de mesure de substance à analyser ;
mesure d'une première température dans le dispositif de mesure de substance à analyser avec un premier capteur de température situé à l'intérieur du boîtier du dispositif de mesure de substance à analyser à proximité de la batterie rechargeable avant le début d'une session de charge ;
détermination si la première température est dans une première plage de température ;
sélection d'un courant de charge maximal élevé pour un courant de charge maximal avant le début d'une session de charge qui ne change pas pendant la session de charge sur la base de la capacité d'une source de charge et si la première température est dans la première plage de température ;
sélection d'un courant de charge maximal réduit pour le courant de charge maximal avant le début de la session de charge qui ne change pas pendant la session de charge sur la base de la capacité de la source de charge et si la première température est en dehors de la première plage de température ;
recharge d'une batterie rechargeable du dispositif de mesure de substance à analyser jusqu'au courant de charge maximal sélectionné ;
déconnexion du câble du dispositif de mesure de substance à analyser et activation du dispositif de mesure de substance à analyser pour réaliser une analyse de substance à analyser ;
insertion d'un élément d'analyse dans un port d'élément d'analyse sur le dispositif de mesure de substance à analyser ;
mesure d'une seconde température avec un second capteur de température situé à l'intérieur du boîtier du dispositif de mesure de substance à analyser à proximité d'un moteur de mesure ;
détermination si la seconde mesure de température est dans une seconde plage de température pour réaliser une analyse de substance à analyser ; et
réalisation de l'analyse de substance à analyser si la seconde température est dans la seconde plage de température,
dans lequel si la première température est inférieure à la première plage de température le courant de charge maximal est réduit pour diminuer un risque de masquage d'un verrouillage faible qui permet une analyse de substance à analyser lorsque la seconde température est supérieure à une valeur la plus faible de la seconde plage de température.

7. Procédé selon la revendication 6, dans lequel si la première température est supérieure à la première plage de température le courant de charge maximal est réduit pour diminuer un risque d'un verrouillage élevé qui empêche une analyse de substance à analyser lorsque la seconde température est supérieure à la seconde plage de température.

8. Procédé selon la revendication 6, dans lequel le courant de charge est sélectionné dans la plage allant d'environ 50 mA à environ 450 mA.

9. Procédé selon la revendication 6, dans lequel l'élément d'analyse a une plage de température allant d'environ 4 °C à environ 45 °C.

10. Procédé selon la revendication 6 dans lequel le dispositif de mesure de substance à analyser portable est sélectionné parmi l'un d'un dispositif de commande de pompe à perfusion portable avec glucomètre et d'un glucomètre portable.
